(19) Europäisches Patentamt
European Patent Office
Office européen des brevets

(11) Publication number: **0 261 073 A2**

# (12) EUROPEAN PATENT APPLICATION

(21) Application number: 87730098.8

(22) Date of filing: 19.08.87

(51) Int. Cl.[4]: **C 07 C 43/29**
C 07 C 43/174,
C 07 D 213/64, A 01 N 31/14,
A 01 N 43/40

(30) Priority: 21.08.86 DE 3628300

(43) Date of publication of application:
23.03.88 Bulletin 88/12

(84) Designated Contracting States:
AT BE CH DE ES FR GB GR IT LI LU NL SE

(71) Applicant: SCHERING AKTIENGESELLSCHAFT Berlin und Bergkamen
Müllerstrasse 170/178 Postfach 65 03 11
D-1000 Berlin 65 (DE)

(72) Inventor: Franke, Heinrich, Dr.
Fabeckstrasse 38
D-1000 Berlin 33 (DE)

Franke, Helga, Dr.
Karl-Stieler-Strasse 2b
D-1000 Berlin 41 (DE)

Krüger, Hans-Rudolf, Dr.
Kulmbacher Strasse 15
D-1000 Berlin 30 (DE)

Baumert, Dietrich, Dr.
Schulzenfdorfer Strasse 108b
D-1000 Berlin 28 (DE)

Joppien, Hartmut, Dr.
Juttastrasse 18
D-1000 Berlin 37 (DE)

## (54) Insecticidal and acaricidal alkane and alkoxy alkane derivatives.

(57) There are provided new alkane and alkoxyalkane derivatives of the general formula I

$$R_1 - \underset{R_2}{\overset{CH_2F}{C}} - CH_2 - A - CH_2 - R_4 \qquad (I)$$

in which
$R_1$ is phenyl, $C_{1-4}$-alkylphenyl, halophenyl, $C_{1-4}$-alkoxyphenyl or halo-$C_{1-4}$-alkoxyphenyl;
$R_2$ is hydrogen, methyl or fluoromethyl;
$R_4$ is phenyl or pyridyl or these groups substituted by one or more of phenoxy or halogen; and
A is $CH_2$ or O,
processes for their preparation and insecticidal and acaricidal compositions containing these compounds.

EP 0 261 073 A2

**Description**

## INSECTICIDAL AND ACARICIDAL ALKANE AND ALKOXY ALKANE DERIVATIVES

The invention relates to new alkane and alkoxyalkane derivatives, processes for their preparation and their use in insecticidal and acaricidal compositions.

It is already known that certain alkane and alkoxyalkane derivatives possess insecticidal and acaricidal properties (DE-OS 31 17 510 and DE-OS 33 17 908).

The object of the present invention is the preparation of new compounds that combat insects and spider mites better than compounds known for this purpose.

The alkane and alkoxyalkane derivatives of the invention are of the general formula I

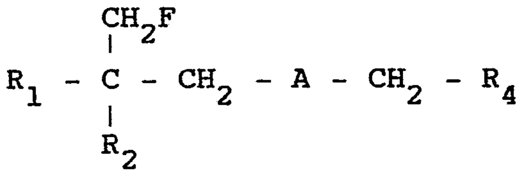
(I)

in which
$R_1$ is phenyl, $C_{1-4}$-alkylphenyl, halophenyl, $C_{1-4}$-alkoxyphenyl or halo-$C_{1-4}$-alkoxyphenyl;
$R_2$ is hydrogen, methyl or fluoromethyl;
$R_4$ is phenyl or pyridyl or these groups substituted by one or more of phenoxy or halogen; and
A is $CH_2$ or O.

The compounds of the invention have insecticidal and acaricidal activity and as a result can be used for combating a wide range of insectes and acarids, including animal ectoparasites. Examples include Lepidoptera, such as Plutella xylostella, Spodoptera littoralis, Heliothis armigera and Pieris brassicae; Diptera, such as Musca domestica, Ceratitis capitata, Erioischia brassicae, Lucilia sericata and Aedes aegypti; Homoptera, including aphids such as Megoura viciae and Nilaparvata lugens; Coleoptera, such as Phaedon cochleariae, Anthonomus grandis, Epilachna varivestis and corn rootworms (Diabrotica spp. eg. Diabrotica undecimpunctata); Orthoptera, such as Blattella germanica; ticks, such as Boophilus microplus and lice, such as Damalinia bovis and Linognathus vituli, as well as spider mites such as Tetranychus urticae and Panonychus ulmi.

The compounds of the invention exist as optional isomers. The invention includes all isomers as well as mixtures of them.

The compounds of the invention, where A is a methylene group, can be prepared, in known manner, by reacting a compound of general formula II

$(R_5)_3\overset{\oplus}{P}\text{-}CH_2\text{-}CH_2\text{-}R_4X^{\ominus}$ (II)

or of general formula III

$(R_6O)_2\overset{\overset{O}{\|}}{P}\text{-}CH_2\text{-}CH_2\text{-}R_4$ (III)

first with a base and then with a compound of general formula IV

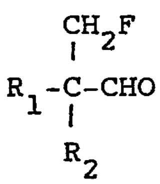
(IV)

to give a compound of general formula V

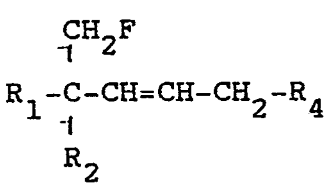
(V)

and then reducing this to the desired product.

The compounds of the invention, where A is oxygen, can be prepared in known manner by reacting a compound of general formula VI

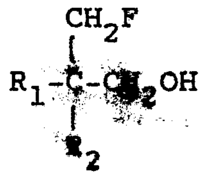 (VI)

with a compound of general formula VII

Z-$CH_2$-$R_4$ (VII)

in the presence of a base and using a solvent, in which $R_1$, $R_2$ and $R_4$ have the meanings given above and Z is halogen, methanesulphonate or toluenesulphonate.

When A = $CH_2$, the reaction is preferably carried out in the presence of an inert solvent, such as that generally used in Wittig reactions. Suitable solvents include alaphatic or aromatic hydrocarbons, such as for example hexane, benzene or toluene, and ethers such as for example diethyl ether and tetrahydrofuran. Other suitable solvents are amides, such as dimethylformamide or hexamethylphosphoric acid triamide. In some cases alcohols or dimethyl sulphoxide can be used.

Suitable bases for the Wittig reaction include metal alcoholates, such as for example sodium ethanolate, metal hydrides, such as for example sodium hydride, metal amides, such as for example sodium amide and organometalic compounds, such as for example phenyllithium or butyllithium.

The compounds of general formula I in which the group $R_1$ is an alkoxyphenyl or haloalkoxyphenyl group, and A = $CH_2$, can also be obtained by treatment of a hydroxyphenyl derivative that can be prepared by hydrolysis of another alkoxyphenyl derivative, for example with the corresponding alkyl halide.

The reaction with compounds of formula VI (etherification) is generally carried out in solution. Suitable bases for the eterification include metal alcoholates, such as for example potassium tert-butylate, metal hydrides, such as for example sodium hydride, metal amides, such as for example lithium diisopropylamide and metal alkyl compounds, such as for example ethyl mangnesium bromide or butyllithium.

Suitable solvents, as opposed to the reactants, especially the bases, include inert substances such as aliphatic and aromatic hydrocarbons such as for example hexane, benzene or toluene and ethers such as for example diethyl ether, tetrahydrofuran or dimethoxyethane. Suitable further amides include dimethylformamide, N-methylpyrrolidone or hexamethylphosphoric acid triamide.

The etherification can further be carried out in a two phase system by using a catalyst and optionally a solvent. Bases that can be used include alkali metal hydroxides or alkali metal carbonates, either as solids or an aqueous solution. Suitable solvents are the reactants themselves as long as they are liquid. Otherwise they can be used in substances which are inert to the bases and which are immiscible with water, such as aliphatic or aromatic hydrocarbons, such as for example hexane, benzene or toluene. Suitable catalysts include crown ethers and quaternary ammonium salts, such are described in Dehmlow and Dehmlow, Phase Transfer Catalysts, Weinheim 1980.

The reaction can be carried out at temperatures between -78 and 140° C, preferably at 20 - 80° C, generally at room temperature.

The necessary starting materials for the preparation of the compounds of the invention are either known or can be prepared in known manner. Thus the aldehyde used as starting material of general formula IV, in which $R_2$ = $CH_3$, can be prepared by reacting a compound of formula XIII

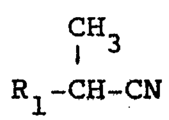 (XIII)

by analogy with hydroxymethlation of the a corresponding phenylpropionic acid ester (G. Schwenker, R. gerber, Chem. Ber. 100, 2460-2461 (1967)), with formaldehyde to give the compound of formula XIV ($R_5$ = OH)

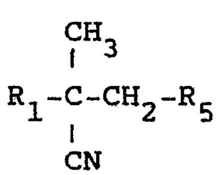 (XIV)

converting the alcohol in known manner to the tosylate or mesylate ($R_5$ = OTos or OMes) and exchanging these leaving groups with potassium fluoride ($R_5$ = F) in ethylene or diethylene glycol. The reduction of the nitrile with aluminium hydrides, such as for example diisobutyl aluminium hydride, to the aldehyde can then be carried out in known manner.

The aldehyde used as starting material of general formula IV, in which $R_2$ = $CH_2F$, can be prepared by

reacting a 4-nitrobenzyl cyanide with formaldehyde to give the compound of formula XV ($R_6$ = OH)

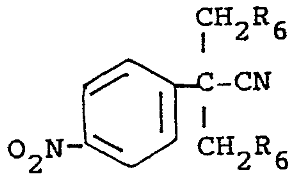

(XV)

converting the diol in known manner to the ditosylate or dimesylate ($R_6$ = OTos or Mes) and exchanging these leaving groups with potassium fluoride ($R_5$ = F) in ethylene or diethylene glycol.

The exchange of the nitro group can be carried out in known manner such as by reduction of the nitro group to an amino group follwed by diazotisation. A halogen can be introduced, for example, by a Sandmeyer reaction, or by heating the diazonium salt and etherification of the corresponding phenol an alkoxy group can be introduced. As the last stage, the reduction of the nitrile with aluminium hydrides, such as for example diisobutyl aluminium hydride, to the desired aldehyde of formula IV can then be carried out in known manner.

The phosphonium salt or phosphonate starting material of general formula II or III can be obtained by treatment of $R_4CH(R_3)CH_2X$ or $R_4CH(R_3)CH_2CH_2X$, wherein X is a halogen atom, with $(R_5)_3P$ or $(R_6O)_3P$.

The alcohols used as starting materials of general formula IV can be prepared by reduction of the corresponding nitrile, aldehyde, carboxylic acid or carboxylic acid ester. The reaction can be carried out according to known methods with metal hydride complexes, for example lithium aluminium hydride or alkyl aluminium hydrides for example diisobutylaluminium hydride. The halide, tosylate and mesylates which are used, are known in themselves or can be prepared according to known methods (DE-OS 31 17 510, DE-OS 33 17 908, Houben-Wevl, Band 5/4, page 354; ibid. Band 9, page 663).

The compounds of the invention prepared by the above described processes can be isolated from the reaction mixture in conventional manner, for example by distillation of the solvent used at normal or reduced pressure or by extraction.

A higher degree of purity can be obtained as general rule by thin layer chromatography purification or by fractional distillation.

The compounds of the invention are, as a rule, colourless oils that are highly soluble in practically all organic solvents but are almost insoluble in water.

The compounds according to the invention can be used at a concentration of 0.0005 to 5%, preferably from 0.001 to 1%, calculated as gram active material per 100ml of the composition.

The compounds of the invention can be used either alone or in mixture with each other or another insecticide. Optionally other plant protection or pesticidal compositions, such as for example insecticides, acaricides or fungicides can be added depending on the desired result.

An improvement in the intensity and speed of action can be obtained, for example, by addition of suitable adjuvants, such as organic solvents, wetting agents and oils. Such additives may allow a decrease in the dose.

Suitable mixture partners may also include phospholipids, e.g. such as from the group phosphatidylcholine, hydrated phosphatidylcholine, phosphatidylethanolamine, N-acyl-phosphatidylethanolamine, phosphatidylinositol, phosphatidylserine, lysolecithin or phosphatidylglycerol.

The designated active ingredients or their mixtures can suitably be used, for example, as powders, dusts, granules, solutions, emulsions or suspensions, with the addition of liquid and/or solid carriers and/or diluents and, optionally, binding, wetting, emulsifying and/or dispersing adjuvants.

Suitable liquid carriers are, for example aliphatic and aromatic hydrocarbons such as benzene, toluene, xylene, cyclohexanone, isophorone, dimethyl sulphoxide, dimethylformamide, other mineral-oil fractions and plant oils.

Suitable solid carriers include mineral earths, e.g. tonsil, silica gel, talcum, kaolin, attapulgite, limestone, silicic acid and plant products, e.g. flours.

As surface-active agents there can be used for example calcium lignosulphonate, polyoxyethylenealkylphenyl ether, naphthalenesulphonic acids and their salts, phenolsulphonic acids and their salts, formaldehyde condensates, fatty alcohol sulphates, as well as substituted benzenesulphonic acids and their salts.

The percentage of the active ingredient(s) in the various preparations can vary within wide limits. For example, the compositions can contain about 10 to 90 percent by weight active ingredients, and about 90 to 10 percent by weight liquid or solid carriers, as well as, optionally up to 20 percent by weight of surfactant.

The agents can be applied in customary fashion, for example with water as the carrier in spray mixture volumes of approximately 100 to 3,000 l/ha. The agents can be applied using low-volume of ultra-low-volume techniques or in the form of so-called microgranules.

The preparation of these formulations can be carried out in a known manner, for example by milling or mixing processes. Optionally, individual components can be mixed just before use for example by the so-called commonly used tank-mixing method.

Formulations can be prepared, for example, from the following ingredients.

a) 80 percent by weight active ingredient
15 percent by weight kaolin
5 percent by weight surface-active agent based on the sodium salt of N-methyl-N-oleyltaurine and the

calcium lignosulphonate
b) 45 percent by weight active ingredient
5 percent by weight sodium aluminium silicate
15 percent by weight cetylpolyglycol ether with 8 moles ethylene oxide
2 percent by weight spindle oil
10 percent by weight polyethylene glycol
23 parts water
c) 20 percent by weight active ingredient
35 percent by weight bentonite
8 percent by weight of the sodium salt of N-methyl-N-oleyltaurine
35 percent by weight silicic acid
d) 20 percent by weight active ingredient
75 percent by weight isophorone
5 percent by weight of an emulsifier mixture of calcium phenylsulphonate and fatty alcohol polyglycol ether

The following examples illustrate the preparation of compounds according to the invention.

Example 1

2-(4-Chlorophenyl)-2-fluoromethylpropyl) 3-phenoxybenzyl ether

Sodium hydride (205 mg; 6.5 mmol) was suspended in dimethoxyethane (20ml) and then in turn, with stirring, there was added 2-(4-chlorophenyl)-2-fluoromethyl-1-propanol (1.25 g; 5.044 mmol), a spatula of sodium iodide and 3-phenoxybenzyl bromide (1.38 g; 5.75 mmol). After stirring for 5 hours at room temperature, the mixture was added to ice-water, extracted 3 times with ether and the extracts washed with water dried over sodium sulphate and evaporated. After chromatography on silica gel using a mixture of ethyl acetate and hexane there was obtained 1.87g of end product (= 84.5% of thoery). $n^{20}D$ : 1.5758

The 2-(4-chlorophenyl)-2-fluoromethyl-1-propanol starting material was prepared as follows:

2-(4-Chlorophenyl)-2-hydroxymethylpropionitrile

To 17.3 g (120.8 mmol) 2-(4-chlorophenyl)propionitrile and 4.49 g (145 mmol) paraformaldehyde in 125 ml dimethyl sulphoxide was added, dropwise, with ice cooling, 12.5 ml 0.5 N of sodium methanolate solution. After 15 min., the reaction mixture was neutralised with glacial acetic acid, diluted with water, extracted with ethyl acetate and the organic phase washed first with sodium hydrogen carbonate solution until neutral and then with aqueous sodium chloride. It was dried over sodium sulphate and concentrated. Distillation at 150°C/0.6 mm gave 16.83 g of the desired product (71.2% of theory).

2-(4-Chlorophenyl)-2-fluoromethylpropionitrile

To 16.83 (86 mmol) 2-(4-chlorophenyl)-2-hydroxymethylpropionitrile in 30 ml pyridine was added, dropwise, with ice-cooling, 6.74 ml (86 mmol) of methanesulphonyl chloride. It was stirred overnight at room temperature, then diluted with methylene chloride, washed with water, dried over sodium sulphate and concentrated. There remained 20.44 g (74.7 mmol, 86.8% of theory) of crude product (mesylate), that was stirred, without further purification, in 200 ml diethyleneglycol with 18.55 g (320.5 mmol) potassium fluoride for one hour at 175°C. After cooling, it was taken up in methylene chloride, washed until neutral with water, dried over sodium sulphate and concentrated. Distillation at 150°C/0.6 mm gave 10.57 g (71.6% of theory).

$^1$H-NMR ($CDCl_3$/TMS, 90 MHz): d= 1,80(d, $^4J_{HF}$= 1,5 Hz; 3H, $CH_3$), AB-System at 4.50 ($J_{AB}$ = 9 Hz; 2H, $CH_2F$): further splitting as d with $^2J_{HF}$ = 48 Hz;7.2 - 7.5 ppm (m; 4H, aromatic-H).

2-(4-Chlorophenyl)-2-fluoromethylpropionaldehyde

To 10.57 g (53.81 mmol) 2-(4-chlorophenyl)-2-fluoromethylpropionitrile in 120 ml absolute toluene, at an inner temperature of 5-10°C, was added dropwise, 56.5 ml of 1.2 molar diisobutylaluminium hydride solution in toluene. After stirring at room temperature for five hours, it was added to ice-water, acidified with dilute hydrochloric acid, extracted with ethyl acetate and the organic phase washed with water and dried over sodium sulphate. The desired aldehyde was obtained, after chromatography on silica gel with a mixture of hexane/acetone (7.0 g = 64.8% of theory).

2-(4-Chlorophenyl)-2-fluoromethyl-1-propanol

2.4 g (12 mmol) 2-(4-chlorophenyl)-2-fluroromethylpropionaldehyde was reduced with sodium borohydride in isopropanol. (Organikum, VEB Deutscher Verlag der Wissenschaften, Berlin 1976, p. 616). After working up, there was obtained 2.3 g (94.8% of theory) of the desired alcohol.

$^1$H/NMR ($CDCl_3$/TMS, 80 MHz): d = 1.33 (d, $^4J_{HF}$ = 2 Hz; 3H, $CH_3$), 1.60 (s (broad); OH), 3.78 (s (broad): 2H, $CH_2OH$), 4.55 (d (broad), $^2J_{HF}$ = 48 Hz; 2H, $CH_2F$), 7.2 - 7.4 ppm (m; 4H, aromatic-H).

Example 2

2-(4-Chlorophenyl)-1-fluoro-2-fluoromethyl-5-(3-phenoxyphenyl)pentane

5.06 g (9.04 mmol) 2-(3-Phenoxyphenyl)ethyltriphenylphosphonium bromide dissolved in 28 ml absolute ether was treated dropwise, with 5.7 ml of a 1.6 molar solution of n-butyllithium in hexane. The mixture was stirred for 90 minutes. 1.85 g (8.5 mmol) 2-(4-chlorophenyl)-3-fluoro-2-fluoromethylpropionaldehyde, dissolved in 5.6 ml ether was added dropwise. After stirring at room temperature for two hours and then standing overnight, the mixture was poured into ice-water, extracted with ether and the organic phase dried and evaporated. After chromatography on silica gel with hexane/ethyl acetate, there was obtained 2.5 g (73.5% of theory) of 2-(4-chlorophenyl)-3-fluoro-2-fluoromethyl-5-(3-phenoxyphenyl)pent-3-ene, as a colourless oil. 1.94 g (4.86 mmol) of this oil, dissolved in 20 ml ethanol/tetrahydrofuran mixture was hydrogenated at room temoerature and atmospheric pressure with the addition of 200 mg palladium catalyst (10% on charcoal). After one hour, the calculated amount of hydrogen had been taken up. The catalyst was removed by filtration and the reaction mixture was chromatographed on silica gel with a mixture of hexane/ethyl acetate. After evaporation there was obtained 1.5 g of product as a colourless oil (77.3% of theory).

n : 1.5494

The 2-(4-chlorophenyl)-3-fluoro-2-fluoromethylpropionaldehyde starting material was prepared as follows:

3-Hydroxy-2-hydroxymethyl-2-(4-nitrophenyl)propionitrile

To a mixture of 500 ml ethanol, 140 ml aqueous formaldehyde (37%) and 100 ml buffer solution (pH 6) (citric acid/aqueous caustic soda; Riedel de Haan AG) was added 100 g (0.062 mmol) solid 4-nitrobenzyl cyanide. The mixture was stirred for 4 hours at 50° C and concentrated on a rotary evaporator. The residue was added to water and treated with ethyl acetate. It was then suction filtered through Celite. After separating the phase the aqueous phase was extracted with ethyl acetate. This extract was dried and evaporated to give 103 g crude product (= 75% of theory), which was used without further purification . After recrystallisation from ether, there was obtained as pure product, m.p. 103-105° C.

3-Methanesulphonyloxy-2-methanesulphonyloxymethyl-2-(4-nitrophenyl)propionitrile

To 103 g (0.465 mol) of the above described crude product and 90.5 ml (1.17 mol) methanesulphonyl chloride, dissolved in 340 ml tetrahydrofuran was added, dropwise at room temperature, 190 ml (2.35 ml) pyridine. The mixture was stirred overnight, poured into ice-water, acidified with concentrated hydrochloric acid and the precipitated crystals filtered off. after drying there remained 138 g (= 78% of theory) of crude product, m.p. 138.5-141° C, which was used without further purification.

3-Fluoro-2-fluoromethyl-2-(4-nitrophenyl)propionitrile

116 g (0.306 mol) of the above described crude dimesylate was dissolved in 700 ml ethyleneglycol and stirred with potassium fluoride for one hour at 175° C. After cooling, the mixture was poured into water, extracted with ethyl acetate and the extract dried and evaporated. The residue was chromatographed on silica gel with a mixture of toluene/ethyl acetate. There was obtained 9.8 g (14.1% of theory) of the desired product as a colourless oil.

$^1$H/NMR ($CDCl_3$/TMS, 90 MHz):

d(ppm) = 4.92 (dd, $^2J_{HF}$ = 46 Hz; $^4J_{HF}$ = 2 Hz; 4 H, $CH_2F$)

7.80 (d, J = 9 Hz, 2 H)

8.33 (d, J = 9 Hz, 2 H)

2-(4-Aminophenyl)-3-fluoro-2-fluoromethylpropionitrile

9.8 g (43.4 mmol) of the above described crude nitrile, dissolved in 100 ml ethanol and 10.7 g tin powder were mixed. 8.5 ml Concentrated hydrochloric acid was added dropwise with stirring. The temperature was maintained below 20° C. The mixture was then heated at 50° C until the reaction was complete. The alcohol was distilled, the residue treated with 30% caustic soda and the precipitate aniline extracted with ether. After washing with water and drying, the extract was evaporated. A dark oil remained which solidified in the refrigerator. Yield: 6.3 g (=74% of theory).

$^1$H/NMR ($CDCl_3$/TMS, 90 MHz):

d(ppm) = 3.80 (s (br); 2 H, $NH_2$)

4.77 (d, J = 46 Hz; 4 H, $CH_2F$)

6.71 (m, 2 H)

7.28 (m, 2 H)

2-(4-Chlorophenyl)-3-fluoro-2-fluoromethylpropionitrile

6.3 g (32 mmol) of the above described crude aniline, was dissolved, with warming, in a mixture of 3.2 ml concentrated hydrochloric acid and 43 ml water. The mixture was then with ice-cooling stirried into a mixture of 3.2 ml concentrated hydrochloric acid and 43 ml water. It was then diazotised at 0-5° C with a solution of of 2.21 g (32 mmol) sodium nitrite in 4.8 ml water. After 10 minutes, excess nitrite was removed by addition of urea and the added dropwise to a mixture of 17.2 ml concentrated hydrochloric acid, 4.26 g (43 mmol) copper (I) chloride and a spatula of copper powder. After stirring at room temperature for an hour, the mixture was poured into water and extracted with ether. The extract was washed with water, dried and evaporated to give 6.0 g of a colourless oil (=87% of theory).

2-(4-Chlorophenyl)-3-fluoro-2-fluoromethylpropionaldehyde

6.0 g (28 mmol) of the above described crude nitrile, was dissolved in 50 ml toluene and treated under a nitrogen atmosphere at room temperature with 27.5 ml diisobutylaluminium hydride (20% solution in toluene, 33 mmol) After stirring for 4 hors, the mixture was added to dilute sulphuric acid and extracted with ethyl acetate. The extract was dried, evaporated and the residue distilled at 130° C/0.6 mm to give a colourless oil. Yield: 3.7 g (=60.7% of theory).

$^1H$/NMR ($CDCl_3$/TMS, 90 MHz):
d(ppm) = 7.15 (d, J = 8.5 Hz; 2 H)
7.43 (d, J = 8.5 Hz; 2 H)
9.40 (t, $^4J_{HF}$ = 3 Hz; 4 H, $CH_2F$)
AB-system at 5.00 ($J_{AB}$ = 10 Hz; 1 H, CHO)
further splitting as dd with
$^2J_{HF}$ = 48 Hz, $^4J_{HF}$ = 1.5 Hz

In a similar way the following compounds were prepared.

| Example | Compound | $n_D^{20}$ |
|---|---|---|
| 3 | 2-(4-Chlorophenyl)-2-fluoromethylpropyl 4-fluoro-3-phenoxybenzyl ether | 1.5641 |
| 4 | 1-Fluoro-2-(4-methoxyphenyl)-5-(3-phenoxyphenyl)pentane | 1.5730 |
| 5 | 2-Fluoromethyl-2-phenylpropyl 4-fluoro-3-phenoxybenzyl ether | 1.5621 |
| 6 | 1-Fluoro-2-fluoromethyl-2-(4-methoxyphenyl)-5-(3-phenoxyphenyl)pentane | 1.5684 |
| 7 | 1-Fluoro-2-fluoromethyl-2-(4-methoxyphenyl)-5-(4-fluoro-3-phenoxyphenyl)pentane | 1.5591 |
| 8 | 2-(4-Ethoxyphenyl)-3-fluoro-2-fluoromethylpropyl 3-phenoxybenzyl ether | 1.5621 |
| 9 | 2-(4-Ethoxyphenyl)-3-fluoro-2-fluoromethylpropyl 4-fluoro-3-phenoxybenzyl ether | 1.5542 |
| 10 | 3-Fluoro-2-fluoromethyl-2-(4-methoxyphenyl)-propyl 3-phenoxybenzyl ether | 1.5643 |
| 11 | 3-Fluoro-2-fluoromethyl-2-(4-methoxyphenyl)-propyl 4-fluoro-3-phenoxybenzyl ether | 1.5536 |
| 12 | 2-(4-Ethoxyphenyl)-1-fluoro-2-fluoromethyl-5-(3-phenoxyphenyl)pentane | 1.5624 |
| 13 | 2-(4-Ethoxyphenyl)-1-fluoro-2-fluoromethyl-5-(4-fluoro-3-phenoxyphenyl)pentane | 1.5530 |

| Example | Compound | $n_D^{20}$ |
|---|---|---|
| 14 | 2-(4-Chlorophenyl)-3-fluoro-2-fluoromethylpropyl 3-phenoxybenzyl ether | 1.5709 |
| 15 | 2-(4-Chlorophenyl)-3-fluoro-2-fluoromethylpropyl 4-fluoro-3-phenoxybenzyl ether | 1.5619 |
| 16 | 2-(4-Chlorophenyl)-3-fluoro-2-fluoromethylpropyl 6-phenoxypyrid-2-ylmethyl ether | 1.5589 |
| 17 | 2-(4-Chlorophenyl)-3-fluoro-2-fluoromethylpropyl pentafluorobenzyl ether | 1.4988 |
| 18 | 1-Fluoro-2-fluoromethyl-2-(4-fluorophenyl)-5-(4-fluoro-3-phenoxyphenyl)pentane | 1.5450 |
| 19 | 2-(4-Bromophenyl)-2-fluoromethylpropyl 4-fluoro-3-phenoxybenzyl ether | 1.5773 |
| 20 | 2-(4-Bromophenyl)-2-fluoromethylpropyl 3-phenoxybenzyl ether | 1.5880 |
| 21 | 2-(4-Bromophenyl)-2-fluoromethylpropyl pentafluorobenzyl ether | m.p.62.5-64°C |
| 22 | 2-(4-Bromophenyl)-2-fluoromethylpropyl 6-phenoxypyrid-2-ylmethyl ether | 1.5720 |
| 23 | 1-Fluoro-2-(4-fluorophenyl)-2-methyl-5-(3-phenoxyphenyl)pentane | 1.5634 |
| 24 | 3-Fluoro-2-(4-fluorophenyl)-2-methyl 3-phenoxybenzyl ether | 1.5612 |

| Example | Compound | $n_D^{20}$ |
|---|---|---|
| 25 | 1-Fluoro-2-(4-fluorophenyl)-2-methyl-5-(4-fluoro-3-phenoxyphenyl)pentane | 1.5504 |
| 26 | 3-Fluoro-2-(4-fluorophenyl)-2-methylpropyl 4-fluoro-3-phenoxybenzyl ether | 1.5515 |
| 27 | 3-Fluoro-2-(4-fluorophenyl)-2-methylpropyl 6-phenoxypyrid-2-ylmethyl ether | 1.5590 |

The following test Examples illustrate the possible uses of the compounds of the invention that have been suitably formulated for use.

Test Example A

Activity in prophylactic treatment of leaves against brown rice-hoppers (Niliparvata lugens Stal)

In a heated greenhouse, rice seedlings (about 15 per pot) were grown until formation of the third leaf and then sprayed until dripping wet with an aqueous preparation containing 0.1% of active material. After drying the sprayed leaves, a transparent cylinder was placed over each pot. 30 Adult brown rice-hoppers (Niliparvata lugens) were introduced into each pot. After 2 days at 26°C in the greenhouse, the amount of dead hoppers was determined. The activity was calculated according to Abbott in comparison with several untreated control pots.

Complete death was reached with the compounds of Examples 1-3, 5-14, and 16-27.

Even at a reduced concentration of 0.0064%, activities of 90% and greater were found with compounds 1, 2, 8, 12 and 13.

Test Example B

Activity in the curative treatment of broad beans (Vicia fabae) against black bean aphids (Aphis fabae scop.)

In a heated greenhouse, seedlings of broad beans (Vicia fabae)(one plant per pot) were grown to a height of about 6 cm. The plants were then covered with a culture of black bean aphid (Aphis fabae). After the each plant had been colonised with 100 to 200 insects, they were each sprayed with the respective active ingredient at the desired concentration in an aqueous preparation until dripping wet and left in the greenhouse at about 24°C. After 2 days the amount of dead aphids was ascertained. The activity was calculated according to Abbott by comparison with untreated controls indicated the level of activity.

At 0.1% compounds of the invention according to Examples 1-22, 25 and 26 gave an activity of at least 70%.

Even at a significantly reduced concentration, good activity was attained; compounds of Examples 7, 12 and 13 showing 90% or greater activity at a concentration of 0.0025%.

Test Example C

Activity in the curative treatment of field beans (Phaseolus vulgaris nanus Aschers.) against motile stages of the two spotted mite (Tetranychus urticae Koch)

In a heated greenhouse, seedlings of field beans were grown to full development of the primary leaf and then covered with bits of leaf infested with Tetranychus urticae. One day later the leaf bits were removed and the plants sprayed with 0.1% aqueous preparation of the active ingredient until dripping wet. After 7 days at 22 to 24°C, the amount of dead motile stages of Tetranychus on treated and untreated plants was ascertained. The activity was calculated according to Abbott.

For the compounds of the invention of Examples 7, 8, 9, 12, 13 18-20 and 23-25 this amounted to 80% or more.

Test Example D

Activity in the curative treatment of field beans (Phaseolus vulgaris nanus Aschers.) against eggs of the two spotted mite (Tetranychus urticae Koch)

In a heated greenhouse, seedlings of field beans were grown to full development of the primary leaf and then treated with adult females of Tetranychus urticae. One day later the plants, carrying the eggs which had benn laid i9n the meantime, were sprayed with 0.1% aqueous preparation of the active ingredient until dripping wet. After 7 days at 22 to 24°C, the amount of dead motile stages of Tetranychus on treated and untreated plants was ascertained. The activity was calculated according to Abbott.

Compounds of the invention of Examples 7, 8, 9, 12, 13, 18-20 and 23-25 showed a greater than 90% activity.

Use Example E

Activity against larvae (L3) of the Mexican bean beetle (Epilachna varivestis)

The compounds of the invention were made up as aqueous emulsions at a concentration of 0.1%. French bean plants (Phaseolus vulgaris) in the primary leaf stage were dipped in the preparations. For each test two plant stems with in total four primary leaves were placed in glass vases filled with water and enclosed in plexiglass cylinders. Then five larvae of the Mexican bean beetle (Epilachna varivestis) at the third larval stage were put in the glass cylinders and kept for three days under extended daylight conditions. The mortality of the larvae after three days indicated the level of activity.

In this experiment, the compounds of Examples 1, 2, 3, 5, 6, 7, 8, 9, 10, 11, 12, 13, 14, 15 and 16 attained a 90 to 100% mortality activity.

Test Example F

Activity against larvae (L2) of the cotton army worm (Spodoptera littoralis)

Compounds of the invention were made up as aqueous emulsions at a concentration of 0.1%. Leaflet pairs of beans (Vicia fabae) as well as 10 larvae (L2) of the cotton army worm (Spodoptera littoralis) per experiment were sprayed with 4 mg spray/$cm^2$ of these preparations in polystyrene petri dishes. The closed petri dishes were left in the laboratory under extended daylight conditions for two days. The % mortality of the larvae after two days indicated the level of activity.

In this experiment, the compounds of Examples 1-3, 6-16, 18-20 and 23-27 produced 90 - 100% activity.

Test Example G

Ovicidal activity against eggs of the cotton army worm (Spodoptera littoralis).

The compounds of the invention were made up as aqueous emulsions at a concentration of 0.1%. One day old eggs that had been laid on filter paper by fertilised female moths were dipped in the preparations until they were completely wet and then placed in closed petri dishes in the laboratory under extended daylight conditions for four days. The % inhibition of hatching of the eggs in comparison with untreated eggs indicates the level of activity.

In this experiment, the following compounds of Examples 1-4, 6-9,11, 13, 15, 18, 19, and 23-27 produced 90 - 100% activity.

Test Example H

Insecticidal activity against Musca domestica

Aliquots of acetone solutions of test compounds at various concentrations were applied to 9 cm diameter filter papers placed in the bottom of 9 cm diameter petri dishes closed by glass lids. After evaporation of solvent, the treated surfaces, together with control treated with acetone alone, were then infested with adult houseflies, (Musca domestica) and held at 22°C for 24 hours. The percentage mortality of the insects was then recorded.

Less than 5% mortality resulted in the control treatments whereas the compounds of Examples 1, 3, 14 and 15 had an $LC_{50}$ of 1000 mg/$m^2$ or less.

Test Example I

Insecticidal activity against Lucilia sericata

1 ml aliquots of an acetone solution containing test compound at various concentrations were applied to cotton wool dental rolls 1 cm x 2 cm, contained in glass vials (2 cm diameter x 5 cm long). After drying, the treated materials were then impregnated with 1ml of nutrient solution, infested with first instar larvae of sheep blowfly (Lucilia sericata), closed by a cotton wool plug and held at 25°C for 24 hours.

For the controls the mortality was $<5\%$ whereas the compounds of Examples 1-3, 6, 8, 14, 15, 23 and 25 had an $LC_{50}$ of 100 ppm or less.

Test Example J

Tickicidal activity against Boophilus microplus

9 cm diameter filter papers were impregnated with 1 ml aliquots of acetone solutions of test compound at various concentrations. The papers were allowed to dry and then folded into envelopes in which cattle tick larvae, (Boophilus microplus) were enclosed and held at 25°C and 80% R.H. for 48 hours. The percentage mortality of tick larvae was then recorded and compared with controls.

The controls gave a mortality of less than 5% whereas compounds of Examples 1-4, 6, 8, 10, 14, 15, 20, 22, 23, 25 and 26 caused 50% mortality at a concentration of 30 ppm or less.

Test Example K

Insecticidal activity against Blattella germanica

Aliquots of acetone solutions of test compounds at various concentrations were applied to glass plates (10 cm x 10 cm). After evaporation of solvent, the treated surfaces, together with controls treated with acetone alone, were then infested with second instar nymphs of the German cockroach, (Blattella germanica), retained on the treated surface within PTFE-coated glass rings 6 cm in diameter and held for 24 hours at 22°C. The percentage mortality of the insects was then recorded.

Less than 5% mortality resulted in the control treatments whereas the compounds of Examples 1-4, 8, 14 and 15 had an $LD_{50}$ of 100 mg/$m^2$ or less.

## Claims

1. Alkane and alkoxyalkane derivatives of general formula I

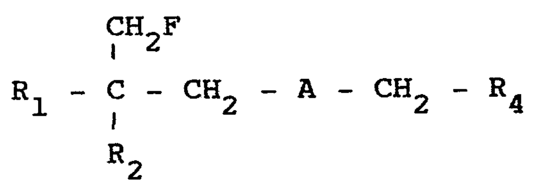

(I)

in which

$R_1$ is phenyl, $C_{1-4}$-alkylphenyl, halophenyl, $C_{1-4}$-alkoxyphenyl or halo-$C_{1-4}$-alkoxyphenyl;

$R_2$ is hydrogen, methyl or fluoromethyl;

$R_4$ is phenyl or pyridyl or these groups substituted by one or more of phenoxy or halogen; and

A is $CH_2$ or O.

2. An insecticidal and acaricidal composition which comprises a compound claimed in claim 1, in admixture with an agriculturally acceptable diluent or carrier.

3. A method of combating insects and acarids which comprises applying to the insects or acarids or their locus an effective amount of a compound claimed in claim 1.